# EUROPEAN PATENT SPECIFICATION

(11) **EP 1 672 082 B1**
(45) Date of publication and mention of the grant of the patent: **16.12.2009**
(21) Application number: 05021784.3
(22) Date of filing: 06.10.2005
(51) Int. Cl.: C12Q 1/68, G01N 33/538

(54) **A method for detecting a target substance using a nucleic acid probe**
Verfahren zum Nachweis einer Zielsubstanz unter Verwendung einer Nukleinsäuresonde
Procédé pour la détection d'une substance cible utilisant une sonde d'acide nucléique

(30) Priority: 08.10.2004 JP 2004296329
(43) Date of publication of application: 21.06.2006
(73) Proprietor: SYSMEX CORPORATION, Kobe-shi, Hyogo 651-0073 (JP)
(72) Inventor: Hasui, Yasushi, Kobe-shi Hyogo 651-0073 (JP); Abe, Shigeki, Kobe-shi Hyogo 651-0073 (JP); Daito, Motonari, Kobe-shi Hyogo 651-0073 (JP); Yamagata, Koichi, Kobe-shi Hyogo 651-0073 (JP)
(74) Representative: HOFFMANN EITLE

(56) References cited:
- WO-A-01/06008
- US-A1- 2002 146 745
- US-A1- 2004 180 369
- US-B1- 6 458 533

## Description

### FIELD OF THE INVENTION

The present invention relates to a method for detecting a target substance in a sample by using a nucleic acid probe, a method of collecting a target substance from a sample, and a reagent kit which can be used in these methods.

### BACKGROUND

As a method for detecting and quantifying a specific target substance, a method of using a highly specific antigen-antibody reaction (immunoassay) is known (JP 2003-107089). According to this immunoassay, a complex of a target substance and an antibody capable of binding to the target substance is bound to an antibody immobilized on a first solidphase and capable of binding to the complex. A substance capable of binding to the antibody immobilized on the first solid phase and different from the complex is competitively reacted therewith, to separate the complex from the first solid phase. The complex is transferred to, and reacted with, a second solid phase wherein an antibody capable of binding to the separated complex is immobilized, whereby the two are bound to each other to detect the target substance in the complex. According to this method, the target substance immobilized on the first solid phase is separated therefrom and transferred to the second solid phase, thus enabling reduction in the background upon detection of the target substance.

However, because the competitive reaction for dissociating the complex from the first solid phase is an equilibrated reaction, not every complex bound to the antibody on the first solid phase is replaced by the counterpart substance in the competitive reaction, so not every target substance can be transferred to the second solid phase. Accordingly, a target substance in a sample containing a large amount of contaminants may not be detected with sufficient accuracy, and there is a need for development of techniques by which a target substance can be detected with higher accuracy.

Under these circumstances, the present invention provides a method of measuring a target substance highly accurately with low background.

### SUMMARY

The present invention provides a method for detecting a target substance, a method for collecting a target substance and a reagent kit for detection of a target substance.

The scope of the present invention is defined solely by the appended claims, and is not affected to any degree by the statements within this summary.

A first aspect of the present invention relates to a method for detecting a target substance. The method for detecting a target substance comprises the steps of:
forming a first complex comprising
   (a) a first nucleic acid probe immobilized on a first solid phase and having a single-strand portion,
   (b) a nucleic acid ligand which has a binding section as a base sequence hybridizable with the single-strand portion of the first nucleic acid probe and which has a target-binding substance capable of binding to the target substance,
   (c) the target substance, and
   (d) a labeling substance capable of binding to the target substance;
separating, from the first complex, a second complex comprising the nucleic acid ligand, the target substance and the labeling substance; wherein the separating step is carried out by strand displacement reaction as a template of the single-strand portion of the first nucleic acid probe with a DNA polymerase with strand displacement activity and nucleotide(s) selected from the group consisting of dATP, dGTP, dTTP and dCTP, and
detecting the target substance on the basis of the labeling substance in the second complex.

A second aspect of the present invention relates to a method for collecting a target substance. The method for collecting a target substance comprises the steps of:
forming a first complex comprising
   (a) a first nucleic acid probe immobilized on a first solid phase and having a single-strand portion,
   (b) a nucleic acid ligand which has a binding section hybridizable with the single-strand portion of the first nucleic acid probe and which has a target-binding substance capable of binding to the target substance,
   (c) the target substance, and
   (d) a labeling substance capable of binding to the target substance;
separating, from the first complex, a second complex comprising the nucleic acid ligand, the target substance and the labeling substance; wherein the separating step is carried out by strand displacement reaction as a template of the single-strand portion of the first nucleic acid probe with a DNA polymerase with strand displacement activity and nucleotide(s) selected from the group consisting of dATP, dGTP, dTTP and dCTP, and
collecting the separated second complex.

The third aspect of the present invention relates to a reagent kit for detection of a target substance. The reagent kit comprises:
a first solid phase on which a first nucleic acid probe having a single-strand portion is immobilized;
a nucleic acid ligand having both a binding section hybridizable with the single-strand portion of the first nucleic acid probe and a target-binding substance capable of binding to the target substance;
a second solid phase on which a second nucleic acid probe having a single-strand portion hybridizable with the binding section of the nucleic acid ligand is immobilized; and
a labeling substance capable of binding to the target substance,further comprising DNA polymerase with strand displacement activity and nucleotide(s) selected from the group of dATP, dGTP, dTTP and dCTP.

### BRIEF DESCRIPTION OF THE DRAWINGS

Figs 1 and 2 show schematic diagrams of one embodiment of the present invention.
Fig 3 shows a schematic diagram of the reaction of Experiment 1.
Fig 4 shows a flow electrochemical measurement system.
Fig 5 shows a graph of the results of Experiment 1.
Fig 6 shows signal levels obtained by subtracting the background noise from the results in Fig. 5.
Fig 7 shows a schematic diagram of the first nucleic acid probe immobilized on the plate C.
Fig 8 shows a schematic diagram of the complex consisting of the first nucleic acid probe, the nucleic acid ligand, M13DNA, and the first substance on the plate C.
Fig 9 shows a schematic diagram of the first complex on the plate C.
Fig 10 shows a schematic diagram of the second complex on the plate D.

### DESCRIPTION OF THE PREFERRED EMBODIMENTS

The method for detecting a target substance according to the present invention comprises:
a step 1 of forming a first complex comprising the following (a) to (d) bound to one another:
   (a) a first nucleic acid probe immobilized on a first solid phase and having a single-strand portion,
   (b) a nucleic acid ligand which has a base sequence (hereinafter, referred to as binding section) hybridizable with the single-strand portion of the first nucleic acid probe and which has a target-binding substance capable of binding to the target substance,
   (c) the target substance, and
   (d) a labeling substance capable of binding to the target substance,
a step 2 of separating, from the first complex, a labeled nucleic acid 1 igand/target substance complex comprising the nucleicacidligand, the target substance and the labeling substance, wherein the separating step is carried out by strand displacement reaction as a template of the single-strand portion of the first nucleic acid probe with a DNA polymerase with strand displacement activity and nucleotide(s) selected from the group consisting of dATP, dGTP, dTTP and dCTP, and
a step 3 of forming a second complex comprising the separated labeled nucleic acid ligand/target substance complex and a second nucleic acid probe immobilized on a second solid phase, and
a step 4 of detecting the target substance on the basis of the labeling substance in the second complex.

The term "nucleic acid" in the present specification includes not only DNA and RNA but also artificially systhesized nucleic acid (referred to hereinafter as artificial nucleic acid) such as PNA (peptide nucleic acid), BNA (bridged nucleic acid), and analogues thereof. The term "base sequence" in the present specification includes not only base sequences of DNA and RNA but also base sequence of artificial nucleic acids.

The first and second solid phases may be those capable of stably immobilizing the first or second nucleic acid probes respectively thereon, and can make use of containers such as a plate, a tube, etc. , a membrane, particles, etc., and specific examples include microplates made of glass or synthetic resin such as polypropylene, polystyrene, etc, a nitrocellulose membrane, latex particles, and magnetic particles.

Examples of the target substance include proteins, nucleic acids, sugars, lipids, hormones, heptenes, toxins, viruses, microorganisms, etc. The "microorganisms" refer to microscopic organisms that cannot be observed with the naked eyes, such as bacteria, protista and chromista.

The nucleic acid ligand includes both a target-binding substance specifically recognizing a target substance and a binding section.

The target-binding substance is selected depending on the type of the target substance, and is not particularly limited insofar as it can specifically bind to a target substance. A base sequence, an antibody, a receptor, and an aptamer can be used as the target-binding substance.

The antibody may be either a polyclonal or monoclonal antibody. The "antibody" in the present invention also includes an antibody fragment and its derivative. Specific examples of the antibody fragment and its derivative include Fab, Fab', F(ab)₂ and sFv fragments (Blazar et al., 1997, J. Immunol., 159: 5821-5833 and Bird et al., 1988, Science, 242: 423-426). Subclasses of the antibody are not limited to IgG and may be IgM, etc.

The binding section may be not only a base sequence of DNA or RNA but also a base sequence of an artificial nucleic acid, but is preferably a base sequence of DNA.

The sequence of the binding section is determined depending on the sequence of the first nucleic acid probe. Specifically, this sequence may a sequence having such complementarity as to form a hybrid with the single-strand portion of the first nucleic acid probe, and is not limited to a sequence having complete complementarity to the single-strand portion of the first nucleic acid probe. The complementarity of the binding section to the single-strand portion of the first nucleic acid probe may be 50% or more because the two are preferably not completely complementary to each other, particularly in order to execute a later dissociation step.

The length of the binding section is preferably a length enough for it to hybridize with the first nucleic acid probe.

When the target-binding substance is an aptamer, the binding section may bind to the aptamer, or a part of the aptamer may be the binding section.

The target-binding substance is preferably bound to the end of the binding section. When the binding section is DNA or RNA, the target-binding substance is bound preferably to the 3' end of the binding section. When the target-binding substance is composed of an aptamer, it is preferable that the binding section is formed at the side of the 5' end of the nucleic acid ligand, while the sequence constituting the aptamer is formed at the side of the 3' end of the nucleic acid ligand. When the target-binding substance is an antibody, the antibody can be bound to the nucleic acid ligand, for example by modifying the 3'-end of the nucleic acid ligand, with a thiol group, then binding it to a linker molecule having a maleimide group and modified with a glutaraldehyde group at the opposite side to the maleimide group, and binding this aldehyde group covalently to an amino group present in the antibody.

The labeling substance is a substance having a label and capable of binding to a target substance.

The labeling substance preferably has a substance capable of binding to a target substance (hereinafter, referred to as the second target-binding substance). As the second target-binding substance, a base sequence, an antibody, a receptor, an aptamer, etc. can be used. The region of a target substance which is recognized by the second target-binding substance is preferably different from the region recognized by the target-binding substance in the nucleic acid ligand.

The type of the label is not particularly limited insofar as the label is a substance capable of detecting the second complex upon binding of the label thereto. Examples of the label include substances emitting a detectable signal and substances catalyzing a reaction generating a product emitting a detectable signal.

Specific examples include radioisotopes such as ³²P and ¹²⁵I, fluorescent dyes such as fluorescein, enzymes such as alkali phosphatase (ALP) and horseradish peroxidase (HRP), fluorescent/luminescent substances such as quantum dots and nano particles, substances having electrochemical activity, such as ferrocene, and electrochemical luminescent substances such as ruthenium and an europium complex.

The labeling substance maybe composed of both a primary substance capable of binding to the second complex and a secondary substance capable of binding to the primary substance and having the label described above. In this case, the primary substance binds to the second complex, and further the secondary substance binds to the primary substance thereby indirectly binding the label to the second complex.

When a fluorescent dye is used as the label, its fluorescence density can be measured by irradiating the reaction solution including the second complex with UV light or the like. When a radioisotope is used as the label, its radioactivity may be measured by using a scintillation counter or the like. When an enzyme is used as the label, a substance formed by enzyme reaction after addition of a substance serving as a substrate of the enzyme may be quantified.

The first and second nucleic acid probes may be those having a base sequence hybridizable with the binding section of the nucleic acid ligand. Each of these probes may be a singe strand or may have a sequence having a single-strand portion and a double-strand portion having 2 base sequences different in length and hybridized with each other, wherein the single-strand portion has a sequence hybridizable with the binding section of the nucleic acid ligand. The sequence of the first nucleic acid probe may be the same as, or different from, the sequence of the second nucleic acid probe. These probes may be those which have a stem loop formed from a single strand and have a single-strand portion having a sequence hybridizable with the nucleic acid ligand. The first nucleic acid probe preferably has a stem loop structure. When the probe has a stem loop structure, it is preferable that the stem part of the probe which has formed a double strand is a 5- to 10-mer, and the single-strand portion is a 15- to 20-mer.

These probes may be immobilized directly or indirectly on a solid phase. The method of indirectly immobilizing the probe includes, for example, a method wherein a specific substance A bound to a solid phase, and a first probe having a substance B capable of binding the substance A to the probe can be used, whereby the probe is immobilized indirectly on the solid phase. As a combination of A and B, a combination of streptavidin and biotin or the like can be used. An amino group may be combined with any of bases constituting the probe in order to immobilize the amino group. When the probe has a stem loop structure, the loop region is preferably immobilized on a solid phase. Alternatively, sequence X and sequence XcY may be used as the probe. The sequence X is a single strand base sequence and the sequence XcY has a base sequence Xc complementary to the sequence X and a base sequence Y hybridizable with the binding section of the nucleic acid ligand. By immobilizing sequence X on a solid phase and hybridizing sequence XcY with sequence X, sequence X and sequence XcY can be used as the first probe immobilized on a solid phase.

Hereinafter, the respective steps for carrying out the method of the present invention will be described.

First, a target substance, a first nucleic acid probe, a nucleic acid ligand and a labeling substance are bound to a first solid phase (reaction container, etc.) to form a first complex (step 1).

Binding of the target substance, the first nucleic acid probe and the nucleic acid ligand can be carried out firstly by binding the first nucleic acid probe and the nucleic acid ligand on the first solid phase and then by adding a sample containing the target substance thereto, thereby binding the target substance to the nucleic acid ligand. Binding of the target substance, the first nucleic acid probe and the nucleic acid ligand can be carried out by binding, to the first nucleic acid probe immobilized on the first solid phase, a "nucleic acid ligand/target substance complex previously formed by binding the nucleic acid ligand" to the target substance.

Binding of the labeling substance to the target substance may be conducted before or after a complex of the target substance, the first nucleic acid probe and the nucleic acid ligand is formed.

It is preferable that after the first complex is formed on the first solid phase, the first solid phase is washed. By doing so, the labeling substance, the nucleic acid ligand, the target substance, the target substance, etc. , which have failed to form the first complex, can be removed from the first solid phase. For washing, a washing solution is preferably used. As the washing solution, a buffer such as TBS and PBS can be used.

Then, the labeled nucleic acid ligand/target substance complex is dissociated from the first complex (step 2).

The dissociation method is a method of using a strand displacement reaction.

This is a method wherein the nucleic acid ligand is separated by extension reaction with the first nucleic acid probe as a template in the presence of a DNA polymerase with strand displacement activity (hereinafter, referred to as polymerase) and dNTPs (nucleiotide(s) selected from dATP, dGTP, dTTP and dCTP).

To use this method, at least the first nucleic acid probe is preferably DNA. When the first nucleic acid probe is a single strand, a primer capable of binding to that part of the first nucleic acid probe which does not hybridize with the binding section is preferably used to serve as a starting point of extension reaction with the polymerase.

When the first nucleic acid probe has a stem loop structure, the single-strand portion of the first nucleic acid probe is preferably at the side of the 5' end. In this case, a 3' end of the first nucleic acid probe is double-stranded and thus capable of serving as a starting point of extension reaction, thus eliminating necessity for use of a primer.

In the extension reaction, the temperature of the first complex can be kept at 37 to 50°C, whereby the polymerase binds to the double-stranded part, followed by linking nucleotides one after another with the 3' end of the first nucleic acid probe (or when a primer is used, the 3' end of the primer) as a starting point. That is, the extension reaction occurs with the single-strand portion of the first nucleotide as a template, while hybridization of the first nucleic acid probe with the nucleic acid ligand is cancelled to separate the nucleic acid ligand.

After this method is used, it is preferable that one or two bases at the 5' end of the binding section of the nucleic acid ligand do not have complementarity to the first nucleic acid probe. Thereby, strand displacement by the extension reaction with the polymerase can easily occur.

When the labeled nucleic acid ligand/target substance complex is separated from the first complex by the above methods, the labeled nucleic acid ligand/target substance complex is collected. Then, the collected labeled nucleic acid ligand/target substance complex is transferred to a second solid phase having a second nucleic acid probe immobilized thereon, and the binding section of the labeled nucleic acid ligand/target substance complex is hybridized with the second nucleic acidprobe to form a second complex (step 3).

After formation of the second complex, the target substance is detected (step 4). The detection in this specification includes not only determination of the presence or absence of the target substance but also quantification of the target substance. When the first nucleic acid probe has a stem loop structure, the end of the stem portion may be linked with the end of the binding section prior to the detection of the target substance. When the first nucleic acid probe and the binding section are DNAs, the two can be linked at the end with each other by using a ligase. When the first nucleic acid probe and the binding section are artificial nucleic acids, the two can be linked at the end with each other by photo-linkage or the like. As a method of linking artificial nucleic acids by photo-linkage, a method described in, for example, U.S. No. 6,593,088 B1 can be used.

The end of the first nucleic acid probe is linked with the end of the binding section, whereby the second complex can be stabilized to prevent the labeled nucleic acid ligand/target substance complex from being separated from the second complex upon washing, etc.

Without conducting the formation of the second complex (step 3), a solution containing the labeled nucleic acid ligand/target substance complex separated from the first complex may be transferred to a container different from the first solid phase, followed by detecting the target substance contained in this solution.

A reagent kit as defined in the appendant claims can be used for carrying out the above-described method for detecting a target substance.

### Examples

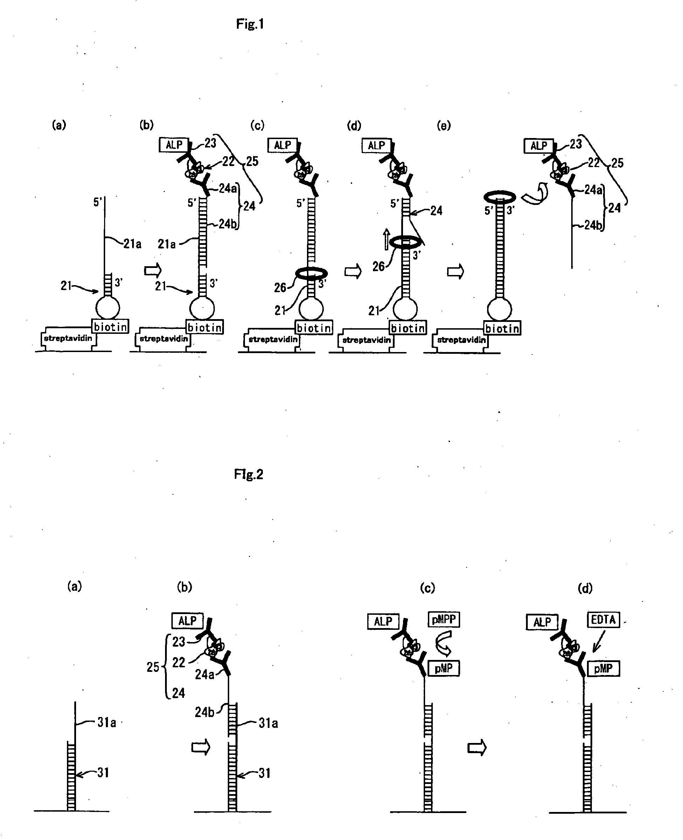

Hereinafter, the method for detecting a target substance according to the present invention will be described in detail by reference to Figs. 1 and 2.

A first nucleic acid probe 21 used in Fig. 1 is a single-stranded DNA and forms a stem loop. A single-strand portion 21a of the first nucleic acid probe has, at the 5' -end thereof, a sequence capable of hybridizing with the binding section of a nucleic acid ligand. Biotin is added to one of bases forming the loop. The first nucleic acid probe includes a probe having a sequence, for example, "AGAACAGCGTATAATCATTAGCCCGCCCGGCCAA*AAAGGCCGGGCGGGCTAA" (SEQ ID NO: 1) . The base "A*" at position 34 is adenine that biotin is added to. The probe of such sequence can be obtained for example by making a request to Takara Bio Co., Ltd. Streptavidin is immobilized at the bottom of a glass container first (solid phase), and biotin of the first nucleic acid probe binds to this streptavidin thereby immobilizing the first nucleic acid probe on the first solid phase (Fig. 1 (a)).

A target substance 22 is a protein (antigen), and a nucleic acid ligand 24 has an antibody which can, as a target-binding substance 24a, bind specifically to this antigen. The nucleic acid ligand 24 has both a binding section 24b ("ATACGCTGTTCT", etc.) as DNA capable of hybridizing with the 5'-end of a single-strand portion 21a of a first nucleic acid probe 21 and the antibody as the target-binding substance 24a bound via a linker molecule to the 3'-end of the binding section 24b.

The target substance 22 has an ALP-labeled secondary antibody 23 bound thereto. When a labeled nucleic acid ligand/target substance complex 25 wherein the target substance having the secondary antibody 23 bound thereto is bound to the target-binding substance 24a is added to the container having the nucleic acid probe 21 immobilized thereon, the binding section 24b of the nucleic acid ligand 24 hybridizes with the single-strand portion 21a of the first nucleic acid probe 21, to form a first complex (Fig. 1 (b)).

When a DNA polymerase 26 with strand displacement activity and dNTPs are added after the first solid phase is washed, the polymerase 26 binds to the 3' end of the first nucleic acid probe 21 (Fig. 1 (c)), and the first nucleic acid probe is extended at the 3' end as a starting point with the sequence, as a template, of the single-strand portion 21a of the first nucleic acid probe 21 (Fig. 1 (d)).

As the extension reaction proceeds, the binding section 24b of the nucleic acid ligand 24 is peeled off to displace the strand. When the single-strand portion 21a of the first nucleic acidprobe 21 becomes double-stranded by the extension reaction, the labeled nucleic acid ligand/target substance complex 25 is completely separated (Fig. 1 (e)).

Then, the labeled nucleic acid ligand/target substance complex 25 obtained by separation is transferred to another container (second solid phase). A second nucleic acid probe 31 is immobilized on the second solid phase (Fig. 2 (a)).

The second nucleic acid probe 31 has double-stranded DNA bound to the solid phase, wherein one strand of the DNA is longer to form a single-strand portion 31a (Fig. 2 (a)). Like the first nucleic acid probe, the second nucleic acid probe 31 may also be a stem loop type having a single-strand portion. The single-strand portion of the second nucleic acid probe is located at the side of the 5' end thereof.

When the nucleic acid ligand/target substance complex 25 is added to the second solid phase having the second nucleic acid probe 31 immobilized thereon, the single-strand portion 31a of the second nucleic acid probe hybridizes with the binding section 24b of the nucleic acid ligand 24, to form a second complex (Fig. 2 (b)). After the second complex is formed, the label of the second complex is measured thereby detecting the target substance 22.

In this example, ALP is used as the label. For example, when a substrate of ALP, for example paramethoxyphenylphosphoric acid (pMPP), is reacted for a predetermined time with ALP in the second complex (Fig. 2 (c)), pMPP is changed into p-methoxyphenol (pMP). After the reaction with ALP is terminated by adding EDTA (Fig. 2 (d)), detectable coloration emitted by pMP can be measured to detect the target substance.

When the above method is used, the background noise generated by adsorption of the labeling substance onto the solid phase without binding thereof to the target substance can be reduced, and thus the target substance can be accurately detected. Dissociation of the target substance from, and capture thereof by, the nucleic acid probe, which occur via the nucleic acid ligand, are based on complementarity to bases and are more reproducible and efficient than those by antigen/antibody reaction, thus enabling highly accurate and highly sensitive detection and measurement. Unlike the antibody, the nucleic acid can be used to determine a base sequence at will to facilitate establishment of the Tm value of the nucleic acid ligand and the nucleic acid probe, thus simplifying the experiment.

The nucleic acid ligand in the present invention is not limited in use to the measurement method of the present invention. For example, the process of from formation of the first complex to dissociation of the nucleic acid ligand/target substance complex from the first complex or to collection of the dissociated nucleic acid ligand/target substance complex is carried out by the measurement method described above, whereby the objective target substance can be separated and collected from a measurement sample containing the target substance. That is, a highly purified and highly concentrated measurement sample can be obtained. Accordingly, the process from the step of forming the nucleic acid ligand/target substance complex to the step of separating and collecting the target substance can be utilized as pretreatment for obtaining a highly purified measurement sample to be subjected to electrophoresis, mass spectrometric analysis, LC, and an intramolecular interaction measurement instrument (SPR-ROM).

### Experiments

### <Experiment 1>

### (1) Immobilization of the first nucleic acid probe

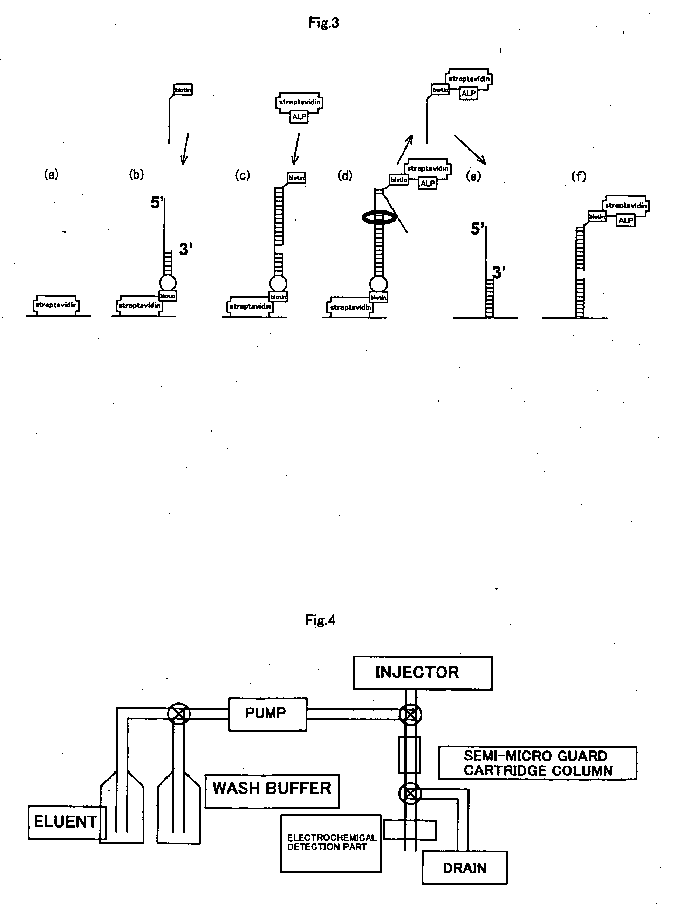

50 µl of 0.2 pmol/µl streptavidin (Chemicon SA101 Lot No. 21080929) was added to each well of a 96-well plate A (ELISA plate manufactured by IWAKI) and left at 37°C for 1. 7 hours tobind streptavidin to the well (Fig. 3 (a)).

After each well of the plate A was washed with TBS (TBS-T) containing 0.1% BSA (manufactured by SIGMA), 300 µl solution prepared by diluting salmon-DNA (salmon sperm DNA) at a concentration of 0.1 mg/ml with the TBS-T was added to each well and left at 37°C for 3 hours to block unspecific adsorption.

DNA for bio-Transfer (DNA forming a stem loop wherein an adenine molecule in the stem region was modified with biotin: 5' AGAACAGCGTATAATCATTAGCCCGCCCGGCCAA*AAAGGCCGGGCGGGCTAA3' (SEQ ID NO: 1)) was used as the first nucleic acid probe. 50 µl of 1 pmol/µl DNA for bio-Transfer was added to each well having streptavidin immobilized thereon, and then left at 37°C for 5 hours. Biotin in the first nucleic acid probe was bound to streptavidin thereby immobilizing the first nucleic acid probe onto the solid phase on each well (Fig. 3 (b)).

### (2) Formation of the first complex

A base sequence having a sequence hybridizable with a single strand of the first nucleic acid probe and containing biotin as the target-binding substance bound to the 3' end thereof (5' ATAGCGCGCTGTTCTTACTCAGGGCACTGCAATTGTGGTCCCAATGGGCTGAGTA 3'-biotin (SEQ ID NO: 2)) was used as the nucleic acid ligand. Bases in positions 1 to 9 in the first nucleic acid probe (SEQ ID NO: 1) and bases in positions 7 to 15 in the nucleic acid ligand are complementary to each other.

The nucleic acid ligand was added to each well and left at room temperature for 1.7 hours thereby hybridizing the nucleic acid ligand with the first nucleic acid probe (Fig. 3 (c)). The amount of the nucleic acid ligand added per 50 µl well was 0 mol, 0.1 × 10⁻¹⁴ mol, 1 × 10⁻¹³ mol, 1 × 10⁻¹² mol, 1 × 10⁻¹¹ mol, or 3.3 × 10⁻¹⁰ mol.

Then, streptavidin (target substance) to which ALP (labeling substance) had been bound was added to each well and captured by biotin of the nucleic acid ligand. Capture of the target substance was carried out by reacting a 2000-fold dilution of ALP-labeled streptavidin (SAv-AP manufactured by NORDIC) with the nucleic acid ligand for 1 hour. Thereby, biotin of the nucleic acid ligand was bound to streptavidin as the target substance, resulting in formation of the first complex.

The amount of the target substance added was such an amount that regardless of the concentration of the nucleic acid ligand in each well, the target substance could bind to every nucleic acid ligand.

### (3) Dissociation step

Reverse Transcriotaqse XL (AMV) manufactured by Takara was used as DNA polymerase with strand displacement activity.

50 µl reaction solution containing the following composition, and 1 µl AMV (5 U/µl), were added to each well and reacted at 37°C for 15 hours.

| | |
|---|---|
| Tris buffer (50 mM Tris-HCl, 40 mM KCl, pH 8.2) | |
| magnesium chloride | 6 mM |
| dNTP | 250 µl |
| DTT | 2 mM |

AMV was bound to the 3' end of the nucleic acid probe forming the first complex, and the single-strand portion of the first nucleic acid probe was used as a template to carry out extension reaction. By this strand displacement reaction, the labeled nucleic acid ligand/target substance complex was dissociated from the first nucleic acid probe (Fig. 3 (d)).

### (4) Immobilization of the second nucleic acid probe

A plate B (GENE PLATE 21GP 02-3 RNAture, Beritus [phonetic]) having oligo-dT20 immobilized thereon was used as the second solid phase.

Single-strand nucleic acid having dA20 and containing a base sequence hybridizable with the nucleic acid ligand was hybridized at room temperature for 1 hour with the plate B to immobilize the second nucleic acid probe (Fig. 3 (e)).

After the plate B was washed, a solution prepared by diluting salmon speruma DNA (containing 3% BSA) at a concentration of 0.01% with TBS-T was added to each well and left at room temperature for 1.5 hours or more for blocking.

### (5) Transfer step

The labeled nucleic acid ligand/target substance complex obtained in (3) was transferred to the plate B having the second nucleic acid probe immobilized thereon. By reaction at room temperature for 1 hour, the single strand of the secondnucleic acidprobe was hybridized with the binding section in the complex, to form the second complex (Fig. 3 (f)).

### (6) Detection step

10 µl solution containing 10 mM p-MPP (diluent: 0.5 M carbonate buffer) as a substrate of ALP was added to each well of the plate B having the second complex formed thereon, followed by ALP reaction at 37°C for 30 minutes. After the ALP reaction, 10 µl terminating solution (EDTA solution, pH 9) was added to each well.

In the reaction solution, p-MPP was decomposed into p-MP by the ALP reaction.

Because the amount of p-MP was changed depending on the number of ALP molecules, the amount of p-MP was measured to determine the amount of the target substance in the second complex.

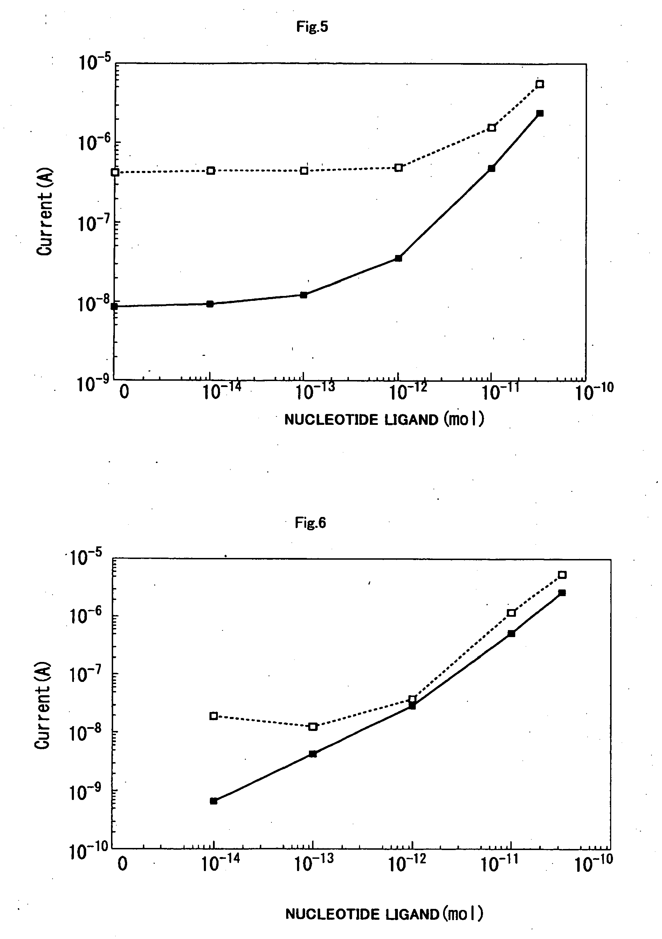

The amount of p-MP was measured by using a flow electrochemical measurement system shown in Fig. 4. The measurement results are shown in a solid-line graph in Fig. 5.

### (Method for detecting the ALP label by an electrochemical method)

The above reaction solution (reaction solution after the ALP reaction) was introduced into an injector. By opening an inject valve (77251 manufactured by Reodine), the reaction solution was passed through an ODS column (YMC-Pack-ODS-AM, AM3C7AM12S05-L502WT No. 0270458 (W)) and then through a short column (YMC semi-micro guard cartridge column 2 × 10, AM12S05-0102CC) and sent to an electrochemical detection part. The eluent used was 0.1 M phosphate buffer containing 45 v/v% or 50 v/v% methanol. The short column, arranged between the inject valve and the electrode detection part, prevented the surface of the electrode from being contaminated with protein components in the measurement sample reaching the electrode detection part. Byusing the short column, the noise electric current derived from the buffer and the electric current of the detected component were separated by the time for reaching the detector [literal trans.].

A degasser (Vacuum Degasser LC-27A) accommodating the eluent removed bubbles from the eluent, and the eluent was sent at a rate of 250 µl/min. via a pump (MICRO LC Pump LC-100) to the electrochemical detection part thereby sending the reaction solution accommodated in the injector to the electrochemical detection part.

The amount of the reaction solution in an FIA-EC-DT (Flow Injection Analysis-Electrochemical-Detection) flow path was predetermined to be 20 µl.

A 3-electrode system was used in the electrode detection part in FIA-EC-DT, wherein Ag/AgCl (BAS RE3V) was used as the reference electrode, a flow path-integrated electrode as the counter electrode, and a glassy carbon electrode (BAS003456) as the work electrode. ALS832a manufactured by BAS was used as the electrochemical measuring instrument.

Each sample was injected into the flow electrochemical measurement system and electrochemically detected by determining a peak of oxidation current. The detection conditions were that the work electrode potential (oxidation potential) was 0.55 V vs Ag/AgCl, and the retention time from the injector to the electrode detection part was 90 seconds.

### <Comparative Example 1>

The procedures (1) to (3) in Experiment 1 were carried out in an the same manner to form a first complex on a 96-well plate. In this state, the ALP reaction was utilized in the same manner as in (6) in Experiment 1, to detect the first complex.

ALP measurement was conducted by using a flow electrochemical detection method shown in Fig. 4. The results are shown in a dotted-line graph in Fig. 5.

Fig. 6 shows signal levels obtained by subtracting the background noise from the results in Fig. 5.

In Figs. 5 and 6, "■" shows the results of Example 1, while "□" shows the results of Comparative Example 1. The amount (mol) of the nucleic acid ligand used (nucleic acid ligand/streptavidin complex) is shown on the abscissa, and the electric current (A) measured in the detection system is shown on the ordinate.

### <Results>

Fig. 5 revealed that in the Comparative Example 1 wherein the dissociation reaction and the transfer of the labeled nucleic acid ligand/target substance complex to the second solid phase were not carried out, the concentration of the target substance was almost correlated with the determined electric current when the concentration of the target substance was 10⁻¹² mol or more, but when the concentration was less than 10⁻¹² mol, the determined electric current was hardly changed. This means that since the background noise is high, samples in a very small amount of less than 10⁻¹² mol cannot be distinguishably measured.

In the Example 1 wherein the dissociation reaction and the transfer of the labeled nucleic acid ligand/target substance complex to the second solid phase were carried out, on the other hand, the concentration of the target substance was almost correlated with the determined electric current. Accordingly, in the assay wherein the dissociation reaction is conduced, a sample can be measured at a concentration of 10⁻¹⁴ mol or at least 10⁻¹³ mol. This means that the detection level was improved by at least one digit as compared with that of conventional assays.

### <Experiment 2>

### (1) Immobilization of the first nuclei acid probe

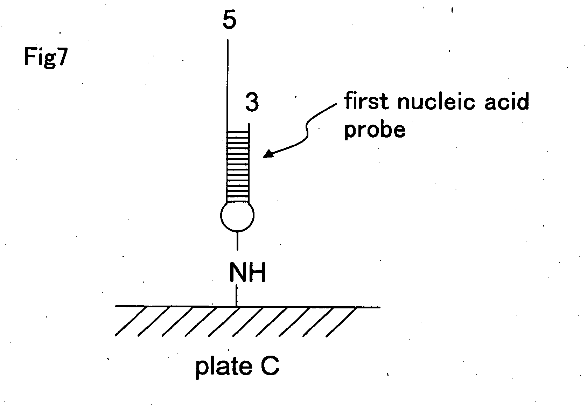

100 µl solution (pH 8.5) containing 50 mM Na₂PO₄, 1 mM EDTA, and 100 pmol aminated oligo DNA (first nucleic acid probe: 5' AGAACAGCGTATAATCATTAGCCCGCCCGGCCAA(NH₂-A)AAGGCCGGGCGGGCTAA 3', SEQ ID NO: 3, Bex Co., Ltd.) was added to each well of a 96-well plate C (DNA-BIND plate, CORNING) and left at 37°C for 1 hour to bind the first nucleic acid probe to the well (Fig. 7).

Each well of the plate C was washed 3 times with 200 µl PBS, and 200 µl blocking solution containing 50 mM Na₂PO₄, 1 mM EDTA and 3% BSA was added to each well and left at 37°C for 30 minutes to block unspecific adsorption.

### (2) Formation of the first complex

As a hybridization buffer for formation of the first complex, a solution prepared by dissolving 5 × SSC (prepared by dilution of 20 × SSC (Nippon Gene)), 0.05% SDS (prepared by dilution of 10% SDS (Nippon Gene)) and 0.005% BSA in ultra-pure water was used.

As the target substance, M13mp18 single-strandDNA (hereinafter, referred to as M13 DNA, manufactured by Takara Bio). This was added to TE buffer (Nippon Gene) containing salmon sperm DNA (Nacalai Tesque) at a concentration of 10 µg/ml, and the prepared solution was used as a measurement sample.

DNA (5' TGACCATACGCTGCTCTTTTTGTAAAACGACGGCCAGT 3', SEQ ID NO: 4, SIGMA Genosys) phosphorylated at the 3' end and having a sequence hybridizable with both the first nucleic acid probe and M13 DNA was used as the nucleic acid ligand.

DNA (5' TTTTTGTCGACTCTAGAGGATC 3' , SEQ ID NO: 5, SIGMA Genosys) biotinated at the 5' end, phosphorylated at the 3' end, and hybridizable with the nucleic acid ligand was used as a first substance for labeling.

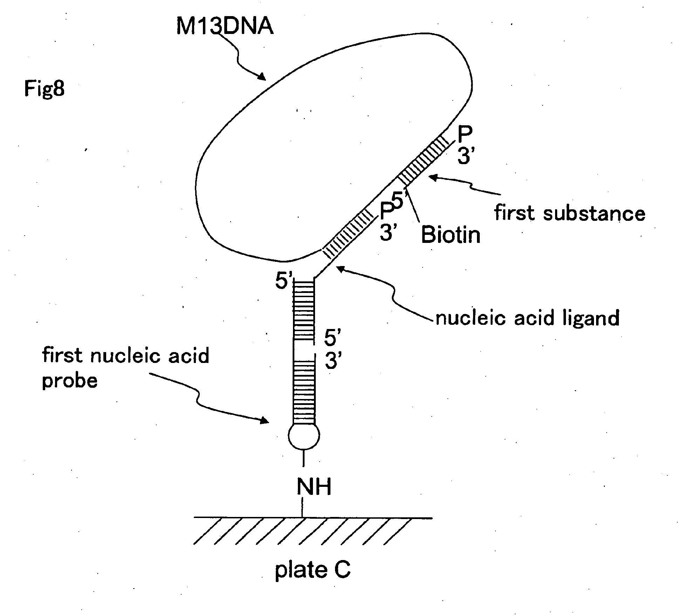

First, 100 µl of the above hybridization buffer was added to each well of the plate C having the first nucleic acid probe immobilized thereon. Then, the measurement sample, 10 pmol nucleic acid ligand and 10 pmol first substance were added to each well of the plate C and left at 37°C for 1 hour, to bind the first probe, the nucleic acid ligand, M13 DNA and the first substance to one another (Fig. 8). Then, each well of the plate C was washed 3 times with 200 µl washing buffer (2 × SSC containing 0.1% SDS).

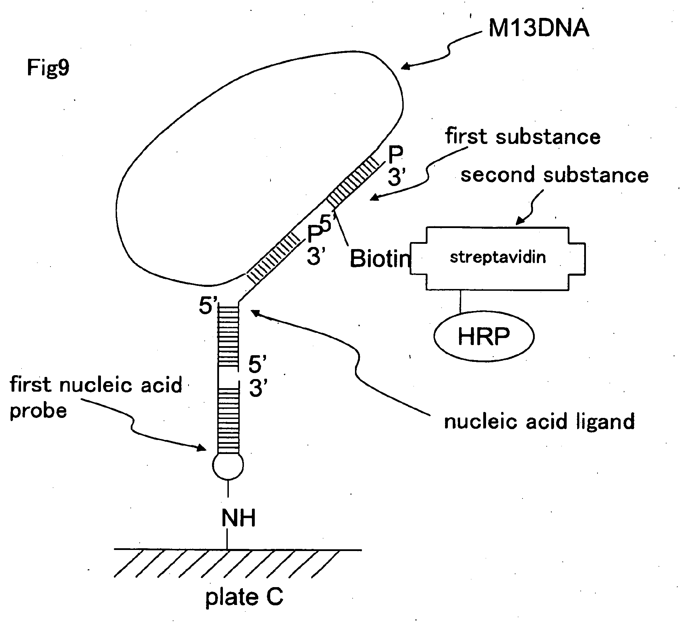

HRP-labeled streptavidin (1000 U/ml, Roche) was used as a second substance for labeling. 200 µl blocking solution (PBS (Nippon Gene) containing 3% BSA) was added to each well of the plate C and left at 37°C for 30 minutes for blocking. After the blocking solution was removed from the well, 100 µl of HRP-labeled streptavidin (1000 U/ml, Roche) diluted 1000-fold with the blocking solution was added to each well and left at 37°C for 1 hour. Thereby, the first complex was formed on the first solid phase (Fig. 9).

### (3) Dissociation step

100 µl reaction solution containing the following composition was added to each well and reacted at 37°C for 1 hour.
1 × Thermopol buffer (New England Labs)
1 mM dATP (Invitrogen)
1 mM dGTP (Invitrogen)
1 mM dTTP (Invitrogen)
1 mM dCTP (Invitrogen)
2.4 U/ml BstDNA polymerase (New England Labs)

BstDNA polymerase is a DNA polymerase with strand displacement activity.

By this reaction, the HRP-labeled nucleic acid ligand/target substance complex was dissociated from the first complex and released into the reaction solution.

### (4) Immobilization of the second nucleic acid probe

100 µl solution (pH 8.5) containing 50 mM Na₂PO₄, 1 mM EDTA, and 100 pmol oligo DNA aminated at the 3' end thereof (second nucleic acidprobe: 5' AGAGCAGCGTATGGTCATTTTTT 3', SEQ IDNO: 6, SIGMAGenosys) was added to each well of a 96-well plate D (DNA-BIND plate, CORNING) and left at 37°C for 1 hour to bind the second nucleic acid probe to the well. Thereafter, the second nucleic acid probe not immobilized on the plate D was removed by washing 3 times with 200 µl PBS.

### (5) Transfer step

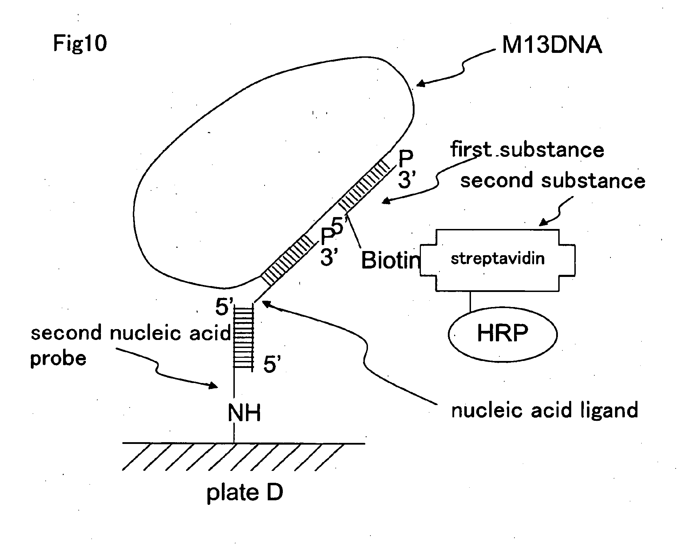

The whole amount of the reaction solution in the plate C obtained in (3) was transferred to each well on the plate D. After transfer, the plate was left at 37°C for 1 hour, whereby the HRP-labeled nucleic acid ligand/target substance complex in the reaction solution was hybridized with the second nucleic acid probe to form the second complex (Fig. 10). After formation of the second complex, each well was washed 3 times with 200 µl PBS.

### (6) Detection step

After the step (5) was finished, PBS for washing each well was removed. Then, 100 µl TMB solution (3,3',5,5'-Tetramethylbensidine Liquid Substrate System Super Sensitive Form for ELISA, SIGMA) was added to each well and reacted at 37°C, and by the action of HRP, the substrate TMB was decomposed and colored. After 1 hour, the reaction was terminated by adding 100 µl of 1 NHCl (Wako Pure Chemical Industries, Ltd.) to each well. The absorbance of each well at 450 nm was measured by a plate reader (Safire 2, TECAN).

For determining the background level in this measurement, (1) to (6) in Experiment 2 were carried out using a target substance-free sample (0 fmol), and the absorbance was determined.

### <Comparative Example 2>

The steps (1) and (2) were carried out in the same manner as in Experiment 2. Without conducting the steps (3) to (5), the coloration reaction with HRP was then conducted in the same manner as in (6), and the absorbance was determined.

For determining the background level in this measurement, the steps (1) and (2) in Experiment 2 were carried out using a target substance-free sample (0 fmol), then the coloration reaction with HRP was conducted in the same manner as in (7), and the absorbance was determined.

### <Results>

The results in Experiment 2 and Comparative Example 2 are shown in Table 1 below.

| amount of target substance | experiment2 | comparative example 2 |
|---|---|---|
| 0 fmol | 0.013 | 0.1405 |
| 10 fmol | 0.1674 | 0.347 |
| S/N ratio | 12.4 | 2.47 |

The background level in Experiment 2 (absorbance in the case where the amount of the target substance was 0 fmol) was about 1/10 relative to the background level in Comparative Example 2. The signal level (absorbance in the case where the amount of the target substance was 10 fmol) in Experiment 2 was about 1/2 relative to the signal level in Comparative Example 2. From the foregoing, the ratio of signal level to background level (noise level) (S/N ratio) was 12.4 in Experiment 2, which was about 5 times as high as the S/N ratio (= 2.47) in Comparative Example 2.

From Experiment 2 and Comparative Example 2, it was confirmed that the background level in detection of the target substance was significantly reduced by separating the labeled nucleic acid ligand/target substance complex from the first solid phase and then transferring it to the second solid phase. The S/N ratio was also improved to enable detection of the target substance with high accuracy.

From the foregoing, a very small amount of the target substance can be measured with high accuracy with low background level according to the detection method of the present invention.

## Claims

1. A method for detecting a target substance, **characterized by** comprising the steps of:
forming a first complex comprising
(a) a first nucleic acid probe immobilized on a first solid phase and having a single-strand portion,
(b) a nucleic acid ligand which has a binding section as a base sequence hybridizable with the single-strand portion of the first nucleic acid probe and which has a target-binding substance capable of binding to the target substance,
(c) the target substance, and
(d) a labeling substance capable of binding to the target substance;
separating, from the first complex, a second complex comprising the nucleic acid ligand, the target substance and the labeling substance; wherein the separating step is carried out by strand displacement reaction as a template of the single-strand portion of the first nucleic acid probe with a DNA polymerase with strand displacement activity and nucleotide (s) selected from the group consisting of dATP, dGTP, dTTP and dCTP, and
detecting the target substance on the basis of the labeling substance in the second complex.

2. The method according to claim 1, comprising the step of forming a third complex comprising the separated second complex and a second nucleic acid probe which is immobilized on a second solid phase and has a single-strand portion hybridizable with the binding section of the nucleic acid ligand,
and wherein the detecting step is carried out by detecting the target substance on the basis of the labeling substance in the second complex included in the third complex.

3. The method according to claim 1 or 2, wherein said target substance is selected from the group consisting of protein, nucleic acid, sugar, lipid, hormone, hapten, toxin, virus and a microorganism.

4. The method according to any one of claims 1 to 3, wherein said first nucleic acid probe, said binding section and said second nucleic acid probe are DNAs.

5. The method according to any one of claims 1 to 4, wherein the binding section is DNA or RNA, and the target-binding substance is bound to the 3' end of the binding section.

6. The method according to any one of claims 1 to 5, wherein said target-binding substance is selected form the group consisting of a base sequence, an antibody, a receptor, and an aptamer.

7. The method according to any one of claims 1 to 6, comprising a step of washing the first solid phase after formation of the first complex.

8. The method according to any one of claims 1 to 7, wherein the formation of the first complex is carried out by binding the target substance and the labeling substance to a complex comprising the first nucleic acid probe and the nucleic acid ligand.

9. The method according to any one of claims 1 to 7, wherein the formation of the first complex is carried out by binding the first nucleic acid probe to a complex comprising the target substance, the target substance bound and the nucleic acid ligand.

10. The method according to any one of claims 1 to 9, wherein said labeling substance is selected from the group consisting of a radioisotope, a fluorescent substance, an enzyme, a coloring substance and a luminescent substance.

11. The method according to claim 10, wherein said labeling substance has a base sequence, an antibody, a receptor, and an aptamer.

12. The method according to any one of claims 1 to 11, wherein the labeling substance comprises a first substance containing a second target-binding substance capable of binding to the target substance, and a second substance capable of binding to the first substance and having a label selected from the group consisting of an enzyme, a fluorescent substance, a luminescent substance, a coloring substance and a radioisotope.

13. A method for collecting a target substance, **characterized by** comprising the steps of:
forming a first complex comprising
(a) a first nucleic acid probe immobilized on a first solid phase and having a single-strand portion,
(b) a nucleic acid ligand which has a binding section hybridisable with the single-strand portion of the first nucleic acid probe and which has a target-binding substance capable of binding to the target substance,
(c) the target substance, and
(d) a labeling substance capable of binding to the target substance;
separating, from the first complex, a second complex comprising the nucleic acid ligand, the target substance and the labeling substance; wherein the separating step is carried out by strand displacement reaction as a template of the single-strand portion of the first nucleic acid probe with a DNA polymerase with strand displacement activity and nucleotide (s) selected from the group consisting of dATP, dGTP, dTTP and dCTP, and
collecting the separated second complex.

14. A reagent kit for detection of a target substance, **characterized by** comprising:
a first solid phase on which a first nucleic acid probe having a single-strand and a double-strand portion and a recessed 3' end is immobilized;
a nucleic acid ligand having both a binding section hybridizable with the single-strand portion of the first nucleic acid probe and a target-binding substance capable of binding to the target substance;
a second solid phase on which a second nucleic acid probe having a single-strand portion hybridizable with the binding section of the nucleic acid ligand is immobilized; and
a labeling substance capable of binding to the target substance; further comprising DNA polymerase with strand displacement activity and nucleotide(s) selected from the group consisting of dATP, dGTP, dTTP and dCTP.

15. The reagent kit according to claim 14, further comprising a washing solution for washing the first solid phase.

## Patentansprüche

1. Verfahren zum Nachweis einer Zielsubstanz, das **dadurch gekennzeichnet ist, dass** es folgende Schritte umfasst:
Bildung eines ersten Komplexes umfassend
(a) eine erste Nukleinsäuresonde, die an einer ersten festen Phase immobilisiert ist und einen einzelsträngigen Teil aufweist,
(b) einen Nukleinsäureliganden, der einen Bindungsabschnitt in Form einer Basensequenz, die mit dem einzelsträngigen Teil der ersten Nukleinsäuresonde hybridisierbar ist, und eine zielbindende Substanz mit der Fähigkeit, an die Zielsubstanz zu binden, aufweist,
(c) die Zielsubstanz und
(d) eine Markierungssubstanz mit der Fähigkeit, an die Zielsubstanz zu binden;
Abtrennung eines zweiten Komplexes, der den Nukleinsäureliganden, die Zielsubstanz und die Markierungssubstanz umfasst, vom ersten Komplex, wobei der Abtrennungsschritt mittels Strangverdrängungsreaktion mit dem einzelsträngigen Teil der ersten Nukleinsäuresonde als Matrize und mit einer DNA-Polymerase mit Strangverdrängungsaktivität und Nukleotid(en) ausgewählt aus der Gruppe bestehend aus dATP, dGTP, dTTP und dCTP durchgeführt wird und
Nachweis der Zielsubstanz anhand der Markierungssubstanz im zweiten Komplex.

2. Verfahren gemäß Anspruch 1, umfassend den Schritt der Bildung eines dritten Komplexes, der den abgetrennten zweiten Komplex und eine zweite Nukleinsäuresonde, die an einer zweiten festen Phase immobilisiert ist und einen einzelsträngigen Teil aufweist, der mit dem Bindungsteil des Nukleinsäureliganden hybridisierbar ist, umfasst, und wobei der Nachweisschritt mittels Nachweis der Zielsubstanz anhand der Markierungssubstanz im zweiten Komplex, der im dritten Komplex inbegriffen ist, durchgeführt wird.

3. Verfahren gemäß Ansprüche 1 oder 2, wobei die Zielsubstanz ausgewählt ist aus der Gruppe bestehend aus Protein, Nukleinsäure, Zucker, Lipid, Hormon, Hapten, Toxin, Virus und einem Mikroorganismus.

4. Verfahren gemäß einem der Ansprüche 1 bis 3, wobei die ersten Nukleinsäuresonde, der Bindungsteil und die zweite Nukleinsäuresonde DNAs sind.

5. Verfahren gemäß einem der Ansprüche 1 bis 4, wobei der Bindungsteil DNA oder RNA ist und die zielbindende Substanz an das 3'-Ende des Bindungsteils gebunden ist.

6. Verfahren gemäß einem der Ansprüche 1 bis 5, wobei die zielbindende Substanz ausgewählt ist aus der Gruppe bestehend aus einer Basensequenz, einem Antikörper, einem Rezeptor und einem Aptamer.

7. Verfahren gemäß einem der Ansprüche 1 bis 6, das einen Waschschritt der ersten festen Phase nach der Bildung des ersten Komplexes umfasst.

8. Verfahren gemäß einem der Ansprüche 1 bis 7, wobei die Bildung des ersten Komplexes mittels Bindung der Zielsubstanz und der Markierungssubstanz an einen Komplex umfassend die erste Nukleinsäuresonde und den Nukleinsäureliganden durchgeführt wird.

9. Verfahren gemäß einem der Ansprüche 1 bis 7, wobei die Bildung des ersten Komplexes mittels Bindung der ersten Nukleinsäuresonde an einen Komplex, der die Zielsubstanz, die gebundene Zielsubstanz und den Nukleinsäureliganden umfasst, durchgeführt wird.

10. Verfahren gemäß einem der Ansprüche 1 bis 9, wobei die Markierungssubstanz ausgewählt ist aus der Gruppe bestehend aus einem Radioisotop, einer fluoreszierenden Substanz, einem Enzym, einem Farbstoff und einer lumineszierenden Substanz.

11. Verfahren gemäß Anspruch 10, wobei die Markierungssubstanz eine Basensequenz, einen Antikörper, einen Rezeptor und ein Aptamer aufweist.

12. Verfahren gemäß einem der Ansprüche 1 bis 11, wobei die Markierungssubstanz eine erste Substanz, die eine zweite zielbindende Substanz mit der Fähigkeit, an die Zielsubstanz zu binden, enthält, und eine zweite Substanz mit der Fähigkeit, an die erste Substanz zu binden, die ein Label ausgewählt aus der Gruppe, bestehend aus einem Enzym, einer fluoreszierenden Substanz, einer lumineszierenden Substanz, einem Farbstoff und einem Radioisotop, aufweist, umfasst.

13. Verfahren zur Gewinnung einer Zielsubstanz, das **dadurch gekennzeichnet ist, dass** es folgende Schritte umfasst:
Bildung eines ersten Komplexes umfassend
(a) eine erste Nukleinsäuresonde, die an einer ersten festen Phase immobilisiert ist und einen einzelsträngigen Teil umfasst,
(b) einen Nukleinsäureliganden, der einen Bindungsabschnitt aufweist, der mit dem einzelsträngigen Teil der ersten Nukleinsäuresonde hybridisierbar ist, und der eine zielbindende Substanz mit der Fähigkeit, an die Zielsubstanz zu binden, aufweist,
(c) die Zielsubstanz und
(d) eine Markierungssubstanz mit der Fähigkeit, an die Zielsubstanz zu binden;
Abtrennung eines zweiten Komplexes, der den Nukleinsäureliganden, die Zielsubstanz und die Markierungssubstanz umfasst, vom ersten Komplex, wobei der Abtrennungsschritt mittels Strangverdrängungsreaktion mit dem einzelsträngigen Teil der ersten Nukleinsäuresonde als Matrize und mit einer DNA-Polymerase mit Strangverdrängungsaktivität und Nukleotid(en) ausgewählt aus der Gruppe bestehend aus dATP, dGTP, dTTP und dCTP durchgeführt wird und
Gewinnung des abgetrennten zweiten Komplex.

14. Reagenskit zum Nachweis einer Zielsubstanz **dadurch gekennzeichnet, dass** es folgendes umfasst:
eine erste feste Phase, an der eine erste Nukleinsäuresonde, die einen einzelsträngigen und einen doppelsträngigen Teil und ein zurückgesetztes 3'-Ende aufweist, immobilisiert ist;
einen Nukleinsäureliganden, der sowohl einen Bindungsteil, der mit dem einzelsträngigen Teil der ersten Nukleinsäuresonde hybridisierbar ist, als auch eine zielbindende Substanz mit der Fähigkeit, an die Zielsubstanz zu binden, aufweist;
eine zweite feste Phase, an die eine zweite Nukleinsäuresonde, die einen einzelsträngigen Teil aufweist, der mit dem Bindungsteil des Nukleinsäureliganden hybridisierbar ist, immobilisiert ist; und
eine Markierungssubstanz mit der Fähigkeit, an die Zielsubstanz zu binden; weiterhin umfassend DNA-Polymerase mit Strangverdrängungsaktivität und Nukleotid(e) ausgewählt aus der Gruppe bestehend aus dATP, dGTP, dTTP und dCTP.

15. Reagenskit gemäß Anspruch 14, weiterhin umfassend eine Waschlösung zum Waschen der ersten festen Phase.

## Revendications

1. Procédé pour détecter une substance cible, **caractérisé par** le fait de comprendre les étapes qui consistent à :
former un premier complexe comprenant :
(a) une première sonde d'acide nucléique immobilisée sur une première phase solide et ayant une partie à brin unique,
(b) un ligand d'acide nucléique qui a une section de liaison comme séquence de bases pouvant être hybridée avec la partie à brin unique de la première sonde d'acide nucléique et qui a une substance de liaison à la cible capable de se lier à la substance cible,
(c) la substance cible, et
(d) une substance de marquage capable de se lier à la substance cible ;
séparer, du premier complexe, un deuxième complexe comprenant le ligand d'acide nucléique, la substance cible et la substance de marquage ; où l'étape de séparation est exécutée par une réaction de déplacement de brins comme modèle de la partie à brin unique de la première sonde d'acide nucléique avec une ADN polymérase avec une activité de déplacement de brins et un/des nucléotide(s) sélectionné(s) dans le groupe constitué de dATP, dGTP, dTTP et dCTP, et
détecter la substance cible sur la base de la substance de marquage dans le deuxième complexe.

2. Procédé selon la revendication 1, comprenant l'étape qui consiste à former un troisième complexe comprenant le deuxième complexe séparé et une deuxième sonde d'acide nucléique qui est immobilisée sur une deuxième phase solide et qui a une partie à brin unique pouvant être hybridée avec la section de liaison du ligand d'acide nucléique, et où l'étape de détection est exécutée en détectant la substance cible sur la base de la substance de marquage dans le deuxième complexe compris dans le troisième complexe.

3. Procédé selon la revendication 1 ou 2, dans lequel ladite substance cible est sélectionnée dans le groupe constitué par une protéine, un acide nucléique, un sucre, un lipide, une hormone, un haptène, une toxine, un virus et un microorganisme.

4. Procédé selon l'une quelconque des revendications 1 à 3, dans lequel ladite première sonde d'acide nucléique, ladite section de liaison et ladite deuxième sonde d'acide nucléique sont des ADN.

5. Procédé selon l'une quelconque des revendications 1 à 4, dans lequel la section de liaison est un ADN ou un ARN, et la substance de liaison à la cible est liée à l'extrémité 3' de la section de liaison.

6. Procédé selon l'une quelconque des revendications 1 à 5, dans lequel ladite substance de liaison à la cible est sélectionnée dans le groupe constitué par une séquence de base, un anticorps, un récepteur, et un aptamère.

7. Procédé selon l'une quelconque des revendications 1 à 6, comprenant une étape de lavage de la première phase solide après formation du premier complexe.

8. Procédé selon l'une quelconque des revendications 1 à 7, dans lequel la formation du premier complexe est exécutée en liant la substance cible et la substance de marquage à un complexe comprenant la première sonde d'acide nucléique et le ligand d'acide nucléique.

9. Procédé selon l'une quelconque des revendications 1 à 7, dans lequel la formation du premier complexe est exécutée en liant la première sonde d'acide nucléique à un complexe comprenant la substance cible, la substance cible liée et le ligand d'acide nucléique.

10. Procédé selon l'une quelconque des revendications 1 à 9, dans lequel ladite substance de marquage est sélectionnée dans le groupe constitué par un radio-isotope, une substance fluorescente, un enzyme, une substance colorante et une substance luminescente.

11. Procédé selon la revendication 10, dans lequel ladite substance de marquage a une séquence de bases, un anticorps, un récepteur, et un aptamère.

12. Procédé selon l'une quelconque des revendications 1 à 11, dans lequel la substance de marquage comprend une première substance contenant une deuxième substance de liaison à la cible capable de se lier à la substance cible, et une deuxième substance capable de se lier à la première substance et ayant un marqueur sélectionné dans le groupe constitué par un enzyme, une substance fluorescente, une substance luminescente, une substance colorante et un radio-isotope.

13. Procédé pour collecter une substance cible, **caractérisé par** le fait de comprendre les étapes qui consistent à :
former un premier complexe comprenant :
(a) une première sonde d'acide nucléique immobilisée sur une première phase solide et ayant une partie à brin unique,
(b) un ligand d'acide nucléique qui a une section de liaison pouvant être hybridée avec la partie à brin unique de la première sonde d'acide nucléique et qui a une substance de liaison à la cible capable de se lier à la substance cible,
(c) la substance cible, et
(d) une substance de marquage capable de se lier à la substance cible ;
séparer, du premier complexe, un deuxième complexe comprenant le ligand d'acide nucléique, la substance cible et la substance de marquage ; où l'étape de séparation est exécutée par une réaction de déplacement de brins comme modèle de la partie à brin unique de la première sonde d'acide nucléique avec une ADN polymérase avec une activité de déplacement de brins et un/des nucléotide(s) sélectionné(s) dans le groupe constitué par dATP, dGTP, dTTP et dCTP, et
collecter le deuxième complexe séparé.

14. Trousse de réactifs pour la détection d'une substance cible, **caractérisée par** le fait de comprendre :
une première phase solide sur laquelle une première sonde d'acide nucléique ayant une partie à brin unique et une partie double brin et une extrémité 3' en retrait est immobilisée.
un ligand d'acide nucléique ayant à la fois une section de liaison pouvant être hybridée avec la partie à brin unique de la première sonde d'acide nucléique et une substance de liaison à la cible capable de se lier à la substance cible ;
une deuxième phase solide sur laquelle une deuxième sonde d'acide nucléique ayant une partie à brin unique pouvant être hybridée avec la section de liaison du ligand d'acide nucléique est immobilisée ; et
une substance de marquage capable de se lier à la substance cible ; comprenant en plus une ADN polymérase avec une activité de déplacement de brins et un/des nucléotide(s) sélectionné(s) dans le groupe constitué par dATP, dGTP, dTTP et dCTP.

15. Trousse de réactifs selon la revendication 14, comprenant en plus une solution de lavage pour laver la première phase solide.


### SEQUENCE LISTING

<110> Sysmex Corporation
<120> A method for detecting a target substance using a nucleic acid probe
<130> 04-015
<160> 6
<170> PatentIn version 3.1
<210> 1
   <211> 52
   <212> DNA
   <213> Artificial
<220>
   <223> Designed DNA
<220>
   <221> stem_loop
   <222> (19).. (52)
   <223>
<400> 1
   agaacagcgt ataatcatta gcccgcccgg ccaaaaaggc cgggcgggct aa 52
<210> 2
   <211> 55
   <212> DNA
   <213> Artificial
<220>
   <223> Designed DNA
<400> 2
   atagcgcgct gttcttactc agggcactgc aattgtggtc ccaatgggct gagta 55
<210> 3
   <211> 52
   <212> DNA
   <213> Artificial
<220>
   <223> Designed DNA
<400> 3
   agaacagcgt ataatcatta gcccgcccgg ccaaaaaggc cgggcgggct aa 52
<210> 4
   <211> 38
   <212> DNA
   <213> Artificial
<220>
   <223> Designed DNA
<400> 4
   tgaccatacg ctgctctttt tgtaaaacga cggccagt 38
<210> 5
   <211> 22
   <212> DNA
   <213> Artificial
<220>
   <223> Designed DNA
<400> 5
   tttttgtcga ctctagagga tc 22
<210> 6
   <211> 23
   <212> DNA
   <213> Artificial
<220>
   <223> Designed DNA
<400> 6
   agagcagcgt atggtcattt ttt 23